# EUROPEAN PATENT SPECIFICATION

(11) **EP 4 197 481 B1**
(45) Date of publication and mention of the grant of the patent: **04.11.2026**
(21) Application number: 22213048.6
(22) Date of filing: 13.12.2022
(51) Int. Cl.: A61B 34/30, A61B 34/00, A61B 17/00

(54) **ROBOTIC SURGICAL ASSEMBLIES INCLUDING SURGICAL INSTRUMENTS HAVING ARTICULATABLE WRIST ASSEMBLIES**
ROBOTISCHE CHIRURGISCHE ANORDNUNGEN MIT CHIRURGISCHEN INSTRUMENTEN MIT BEWEGLICHEN HANDGELENKANORDNUNGEN
ENSEMBLES CHIRURGICAUX ROBOTIQUES COMPRENANT DES INSTRUMENTS CHIRURGICAUX AYANT DES ENSEMBLES POIGNETS ARTICULABLES

(30) Priority: 14.12.2021 US 202163289361 P; 02.11.2022 US 202217979105
(43) Date of publication of application: 21.06.2023
(73) Proprietor: Covidien LP, Mansfield, MA 02048 (US)
(72) Inventor: Heiliger, Zachary S., Boulder, 80301 (US)
(74) Representative: Zimmermann & Partner Patentanwälte mbB

(56) References cited:
- US-A1- 2018 055 589
- US-A1- 2020 229 833
- US-A1- 2020 237 455

## Description

### CROSS-REFERENCE TO RELATED APPLICATION

This application claims the benefit of and priority to U.S. Provisional Patent Application No. 63/289,361, filed December 14, 2021.

### BACKGROUND

Some surgical robotic systems include a console supporting a surgical robotic arm and a surgical instrument or at least one end effector (e.g., forceps or a grasping tool) mounted to the robotic arm. The robotic arm provides mechanical power to the surgical instrument for its operation and movement. Each robotic arm may include an instrument drive unit operatively connected to the surgical instrument and coupled to the robotic arm via a rail. In operation, the robotic arm is moved to a position over a patient and then guides the surgical instrument into a small incision via a surgical trocar or a natural orifice of a patient to position the end effector at a work site within the patient's body. The instrument drive unit drives a rotation of each corresponding driven member of the attached surgical instrument to perform a surgical treatment. The instrument drive unit may be configured to articulate the end effector in a plurality of directions to adjust its pitch and/or yaw within a surgical site, to open/close jaw members, and/or to fire features thereof.

US 2018/055589 A1, US 2020/229833 A1 and US 2020/237455 A1 provide relevant prior art.

### SUMMARY

In accordance with an aspect of the disclosure, a surgical instrument of a surgical robotic system is provided and includes a housing, a first transmission disposed within the housing, a shaft extending distally from the housing, an end effector pivotably coupled to a distal end portion of the shaft, and first and second articulation cables. The first transmission includes a rotatable first input shaft, first and second output shafts configured to rotate in response to a rotation of the first input shaft, and a first differential gear mechanism operably coupling the first input shaft to the first and second output shafts and the first and second output shafts to one another. Each of the first and second articulation cables has a proximal end portion operably coupled to the respective first and second output shafts, and a distal end portion secured to the end effector. The first and second articulation cables move axially in opposing directions at a different rate from one another to articulate the end effector relative to the shaft in response to the rotation of the first input shaft.

In aspects, the first transmission may further include first and second articulation nuts operably coupled to the respective first and second output shafts. The articulation nuts mayb e configured to translate along the respective first and second output shafts in response to a rotation of the first and second output shafts. The articulation cables may be axially fixed to the respective first and second articulation nuts.

In aspects, the first differential gear mechanism may include a ring gear, first and second spider gears coupled to the ring gear, and first and second side axles. The ring gear may be operably coupled to the first input shaft such that the ring gear is configured to be rotated by the first input shaft. The first and second spider gears may be configured to rotate about a respective axis thereof relative to the ring gear and with the ring gear around a rotation axis of the ring gear. The first side axle may be operably coupled to the first output shaft and the first and second spider gears, and the second side axle may be operably coupled to the second output shaft and the first and second spider gears.

In aspects, the first side axle may have opposing first and second gears. The first gear may be operably coupled to the first and second spider gears and the second gear may be operably coupled to the first output shaft.

In aspects, the second side axle may have opposing first and second gears. The first gear of the second side axle may be operably coupled to the first and second spider gears and the second gear of the second side axle may be operably coupled to the second output shaft.

In aspects, the first input shaft may have a pinion gear non-rotationally fixed to a distal end portion thereof. The pinion gear may be in meshing engagement with the ring gear.

In aspects, the ring gear may rotate about an axis that is perpendicular to a rotation axis of the first input shaft.

In aspects, the surgical instrument may further include a second transmission nested with or positioned adjacent the first transmission and supported in the housing. The second transmission may include a rotatable second input shaft, third and fourth output shafts configured to rotate in response to a rotation of the second input shaft, and a second differential gear mechanism operably coupling the second input shaft to the third and fourth output shafts and the third and fourth output shafts to one another.

In aspects, the surgical instrument may further include third and fourth articulation cables each having a proximal end portion operably coupled to the respective third and fourth output shafts, and a distal end portion secured to the end effector, such that the third and fourth articulation cables move axially in opposing directions at a different rate from one another to articulate the end effector relative to the shaft in response to the rotation of the second input shaft.

In aspects, the surgical instrument may further include a wrist assembly movably coupling the end effector to the distal end portion of the shaft. The wrist assembly may be configured to allow the end effector to articulate relative to the distal end portion of the shaft to adjust both a pitch and yaw of the end effector.

In aspects, the first transmission may be configured to change the pitch of the end effector, and the second transmission may be configured to change the yaw of the end effector.

In accordance with another aspect of the disclosure, a surgical robotic system is provided that includes a surgical robotic arm, an instrument drive unit configured to be supported on the surgical robotic arm, and a surgical instrument configured to be coupled to and driven by the instrument drive unit. The surgical instrument includes a housing configured to be attached to the instrument drive unit, a first transmission disposed within the housing, a shaft extending distally from the housing, an end effector pivotably coupled to a distal end portion of the shaft, and first and second articulation cables. The first transmission includes a rotatable first input shaft drivingly coupled to a corresponding drive shaft of the instrument drive unit, first and second output shafts configured to rotate in response to a rotation of the first input shaft, and a first differential gear mechanism operably coupling the first input shaft to the first and second output shafts and the first and second output shafts to one another. Each of the first and second articulation cables has a proximal end portion operably coupled to the respective first and second output shafts, and a distal end portion secured to the end effector, such that the first and second articulation cables move axially in opposing directions at a different rate from one another to adjust a pitch or a yaw of the end effector relative to the shaft in response to the rotation of the first input shaft.

In aspects, the surgical instrument may further include a second transmission nested with or positioned adjacent the first transmission and supported in the housing. The second transmission may further include a rotatable second input shaft, third and fourth output shafts configured to rotate in response to a rotation of the second input shaft, and a second differential gear mechanism operably coupling the second input shaft to the third and fourth output shafts and the third and fourth output shafts to one another. The surgical instrument may further include third and fourth articulation cables each having a proximal end portion operably coupled to the respective third and fourth output shafts, and a distal end portion secured to the end effector, such that the third and fourth articulation cables move axially in opposing directions at a different rate from one another to articulate the end effector relative to the shaft in response to the rotation of the second input shaft.

In aspects, the surgical instrument may further include a wrist assembly movably coupling the end effector to the distal end portion of the shaft. The wrist assembly may be configured to allow the end effector to articulate relative to the distal end portion of the shaft to adjust both the pitch and yaw of the end effector.

In aspects, the first transmission may be configured to change the pitch of the end effector, and the second transmission may be configured to change the yaw of the end effector.

In aspects, the first transmission may further include first and second articulation nuts operably coupled to the respective first and second output shafts and configured to translate therealong in response to a rotation of the respective first and second output shafts. The first and second articulation cables may be axially fixed to the respective first and second articulation nuts.

In accordance with further aspects of the disclosure, a surgical instrument of a surgical robotic system is provided that includes a housing, a first transmission disposed within the housing, a shaft extending distally from the housing, an end effector pivotably coupled to a distal end portion of the shaft, and first and second articulation cables. The first transmission includes a rotatable first input shaft, first and second outputs configured to rotate in response to a rotation of the first input shaft, and a first differential gear mechanism operably coupling the first input shaft to the first and second outputs and the first and second outputs to one another. The first and second articulation cables each have a proximal end portion operably coupled to the respective first and second outputs, and a distal end portion secured to the end effector. The first and second articulation cables move axially in opposing directions at a different rate from one another to change the pitch or yaw of the end effector relative to the shaft in response to the rotation of the first input shaft.

In aspects, each of the first and second outputs may include an articulation wheel configured to rotate via the first differential gear mechanism. The first and second articulation cables may be attached to the respective articulation wheels, such that the rotation of the articulation wheels axially moves the first and second articulation cables in the opposing directions.

In aspects, the first output may include a left-handed lead screw and the second output may include a right-handed lead screw.

In aspects, the first transmission may further include first and second articulation nuts operably coupled to the respective left-handed and right-handed lead screws. The articulation nuts may be configured to translate along the left-handed and right-handed lead screws in response to the rotation of the respective lead screws. The articulation cables may be axially fixed to the respective first and second articulation nuts.

In aspects, the surgical instrument may further include a second transmission nested with or positioned adjacent the first transmission and supported in the housing. The second transmission may include a rotatable second input shaft, third and fourth outputs configured to rotate in response to a rotation of the second input shaft, and a second differential gear mechanism operably coupling the second input shaft to the third and fourth outputs and the third and fourth outputs to one another. The surgical instrument may further include third and fourth articulation cables each having a proximal end portion operably coupled to the respective third and fourth output shafts, and a distal end portion secured to the end effector, such that the third and fourth articulation cables move axially in opposing directions at a different rate from one another to articulate the end effector relative to the shaft in response to the rotation of the second input shaft.

Further details and aspects of exemplary embodiments of the disclosure are described in more detail below with reference to the appended figures.

The invention is defined in appended independent claims 1 and 13, preferred embodiments are defined in appended dependent claims.

As used herein, the terms parallel and perpendicular are understood to include relative configurations that are substantially parallel and substantially perpendicular up to about + or - 10 degrees from true parallel and true perpendicular.

### BRIEF DESCRIPTION OF THE DRAWINGS

Embodiments of the disclosure are described herein with reference to the accompanying drawings, wherein:
FIG. 1 is a schematic illustration of a robotic surgical system in accordance with the disclosure;
FIG. 2 is a perspective view of a surgical robotic arm of the robotic surgical system of FIG. 1 illustrating a surgical instrument and an instrument drive unit being coupled to the surgical robotic arm;
FIG. 3 is a perspective view, with parts shown schematically, of the surgical instrument of FIG. 2;
FIG. 4 is an enlarged, perspective view of a wrist assembly of the surgical instrument shown in FIG. 3;
FIG. 5A is a perspective view illustrating a first transmission of the surgical instrument of FIG. 2;
FIG. 5B is a perspective view illustrating a second transmission of the surgical instrument of FIG. 2; and
FIG. 6 is a perspective view illustrating a third transmission of the surgical instrument of the robotic surgical system of FIG. 2.

### DETAILED DESCRIPTION

Embodiments of the disclosed robotic surgical system and methods thereof are described in detail with reference to the drawings, in which like reference numerals designate identical or corresponding elements in each of the several views. As used herein the term "distal" refers to that portion of the robotic surgical system or component thereof that is further from the user, while the term "proximal" refers to that portion of the robotic surgical system or component thereof that is closer to the user.

Articulation in a wristed robotic instrument is not linear. Stated differently, the wrist imposes a non-linear curve into the system. Consequently, an inside articulation cable is pulled back further than an outside articulation cable, therefore covering a greater axial distance in the same amount of time. Thus, because the two articulation cables are not driven the same distance during the same amount of time, the disclosure provides a differential. The differential converts rotational motion of an input shaft to rotational motion of two output axles. An open differential system allows for torque to be applied regardless of rotational differences between the output axles, covering variable distances in the same amount of time.

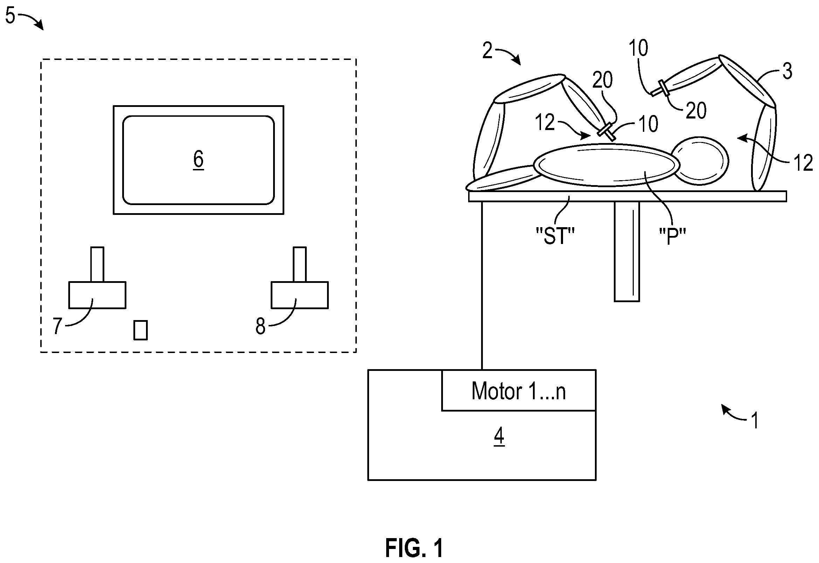

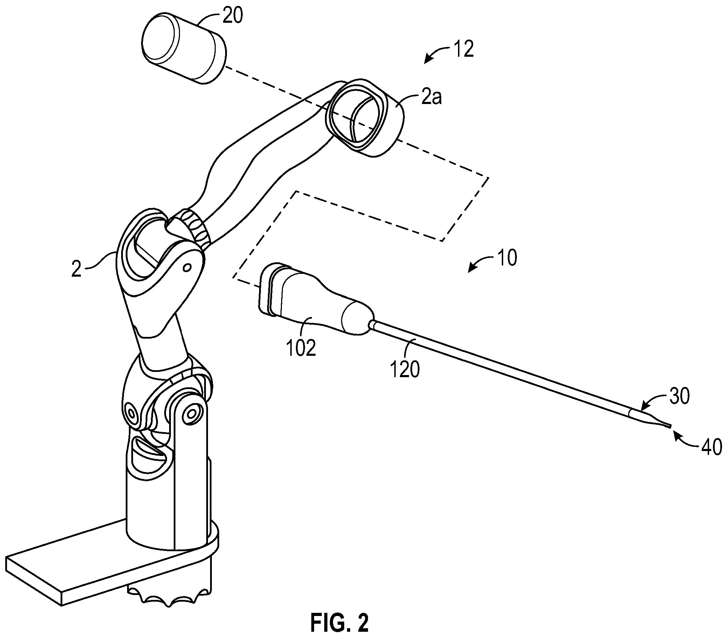

Referring initially to FIGS. 1 and 2, a robotic surgical system 1 is shown and generally includes a plurality of surgical robotic arms 2, 3 each having a surgical instrument 10 (e.g., an electrosurgical instrument, a surgical stapling instrument, a surgical forceps, or the like) removably coupled thereto; a control device 4 (e.g., a computer); and an operating console 5 coupled with the control device 4.

With continued reference to FIG. 1, the operating console 5 includes a display device 6, which is set up to display two-dimensional and three-dimensional images; and manual input devices 7, 8 that serve to enable a user (e.g., a surgeon) to telemanipulate robotic arms 2, 3, as known in principle to a person skilled in the art. Each of the robotic arms 2, 3 may include a plurality of members that are interconnected by joints. The robotic arms 2, 3 may be driven by electric drives (not shown) that are connected to the control device 4. The control device 4 is set up to execute a computer program to activate the electric drives in such a way that the robotic arms 2, 3, their instrument drive units 20, and thus the surgical instrument 10 execute a movement in accordance with a movement of the manual input devices 7, 8. The control device 4 may also be set up in such a way that it regulates the movement of the robotic arms 2, 3 and/or of the electric drives.

The robotic surgical system 1 is configured for minimally invasive treatment of a patient "P" lying on a surgical table "ST" using a surgical instrument (e.g., surgical instrument 10) coupled to the robotic surgical system 1. In some embodiments of the disclosure, the robotic surgical system 1 may include more than two robotic arms that are likewise coupled to the control device 4 and telemanipulatable by the operating console 5. A surgical instrument (e.g., surgical instrument 10) may also be attached to the additional robotic arm(s).

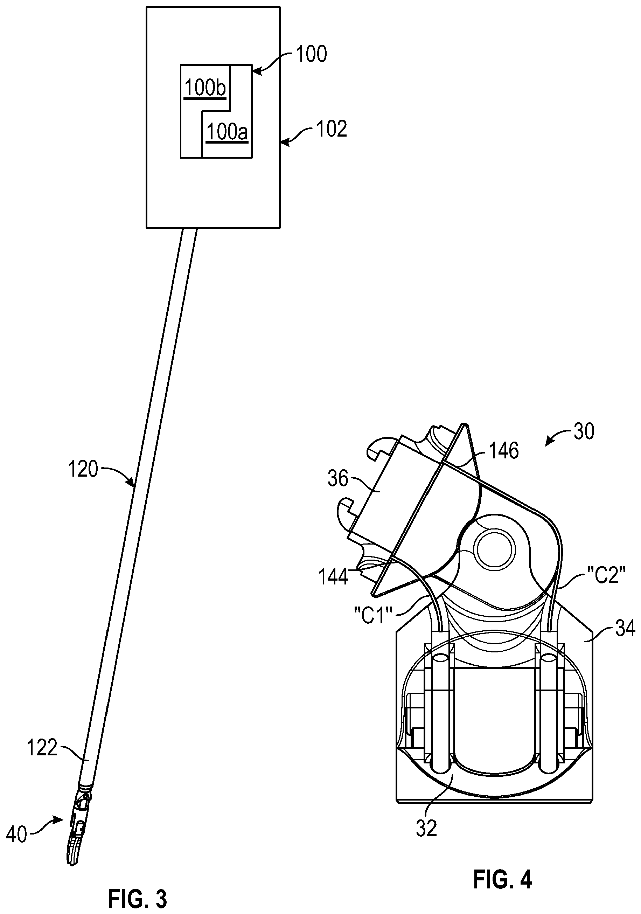

The surgical instrument 10 includes an end effector 40 (FIG. 2) for grasping and, in aspects, treating tissue. The control device 4 may control a plurality of motors (Motor 1...n) with each motor configured to drive a relative rotation of drive members of a transmission assembly 100 (FIG. 3) of the surgical instrument 10 to effect operation and/or movement of the end effector 40 of the surgical instrument 10. It is contemplated that the control device 4 coordinates the activation of the various motors (Motor 1...n) to coordinate a clockwise or counter-clockwise rotation of drive members (not shown) of the instrument drive unit 20 in order to coordinate an operation and/or movement of the end effector 40. In embodiments, each motor can be configured to actuate a drive rod or a lever arm to effect operation and/or movement of the end effector 40 of the surgical instrument 10.

With specific reference to FIG. 2, the robotic surgical system 1 includes a surgical assembly 12, which includes the robotic arm 2, the surgical instrument 10 coupled to the robotic arm 2, and the instrument drive unit 20 configured to operably couple to the surgical instrument 10. The instrument drive unit 20 is configured for powering the surgical instrument 10. The instrument drive unit 20 transfers power and actuation forces from its motors (not shown) to the transmission assembly 100 (FIG. 3) of the surgical instrument 10 to ultimately drive movement of components of the end effector 40, for example, a movement of a knife blade (not explicitly shown) for cutting tissue and a closing and opening of jaw members of the end effector 40 for grasping tissue, and/or drive an articulation of the end effector 40.

With reference to FIGS. 2 and 3, the surgical instrument 10 generally includes a housing 102, a shaft 120 extending distally from the housing 102, and a wrist assembly 30 pivotably coupling the end effector 40 to the shaft 120. The housing 102 is configured to hook, latch, or otherwise attach to a surface of the robotic arm 2, e.g., the distal end 2a of the robotic arm 2, to secure the surgical instrument 10 to the robotic arm 2. In embodiments, the housing 102 may be attached to the surgical robotic arm 2 via various fastening engagements, such as, for example, clips, latches, friction fit engagement, buttons, a variety of fasteners, and/or a bayonet-type connection. The housing 102 houses the transmission assembly 100 that interfaces with the instrument drive unit 20. The transmission assembly 100 translates the motion and torques of the motors of the instrument drive unit 20 into the motion necessary to articulate the wrist assembly 30 of the surgical instrument 10, open and close the jaw members of the end effector 40, and deploy and retract a knife blade to cut tissue grasped between the jaw members of the end effector 40.

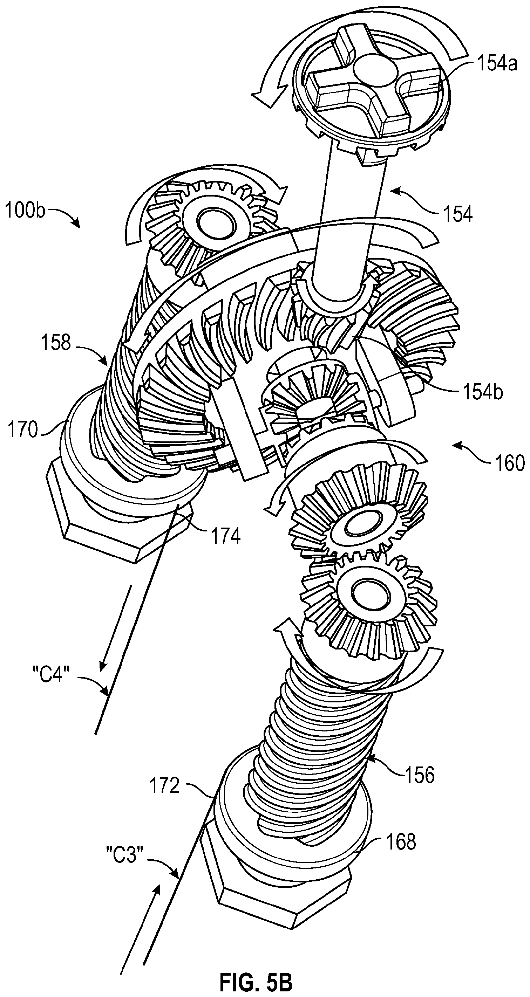

With brief reference to FIG. 4, the wrist assembly 30 of the surgical instrument 10 operably couples the end effector 40 to a distal end portion 122 (FIG. 3) of the shaft 120. More specifically, the wrist assembly 30 has a proximal body 32 immovably attached to the distal end portion 122 of the shaft 120, a proximal pivot member 34 pivotably coupled to the proximal body 32, and a distal pivot member 36 pivotably coupled to the proximal pivot member 34. The proximal pivot member 34 is pivotable about a first articulation axis relative to the proximal body 32 to adjust a pitch of the end effector 40, and the distal pivot member 36 is pivotable relative to the proximal pivot member 34 about a second articulation axis to adjust a yaw of the end effector 40. The wrist assembly 30 is configured to affect the pivoting motion of the end effector 40 relative to the shaft 120 to adjust the yaw and/or pitch of the end effector 40 utilizing a series of translatable cables driven by the motors of the instrument drive unit 20. One set of articulation cables "C1," "C2" for adjusting the yaw of the end effector 40 are routed through the wrist assembly 30 and fixed to the distal pivot member 36 of the wrist assembly 30 or a proximal end of the end effector 40. Accordingly, translation of selected cables pivots the end effector 40 in one of a plurality of directions, as will be described further below. The cables "C3," "C4" for effecting the change in pitch of the end effector 40 are shown in FIG. 5B.

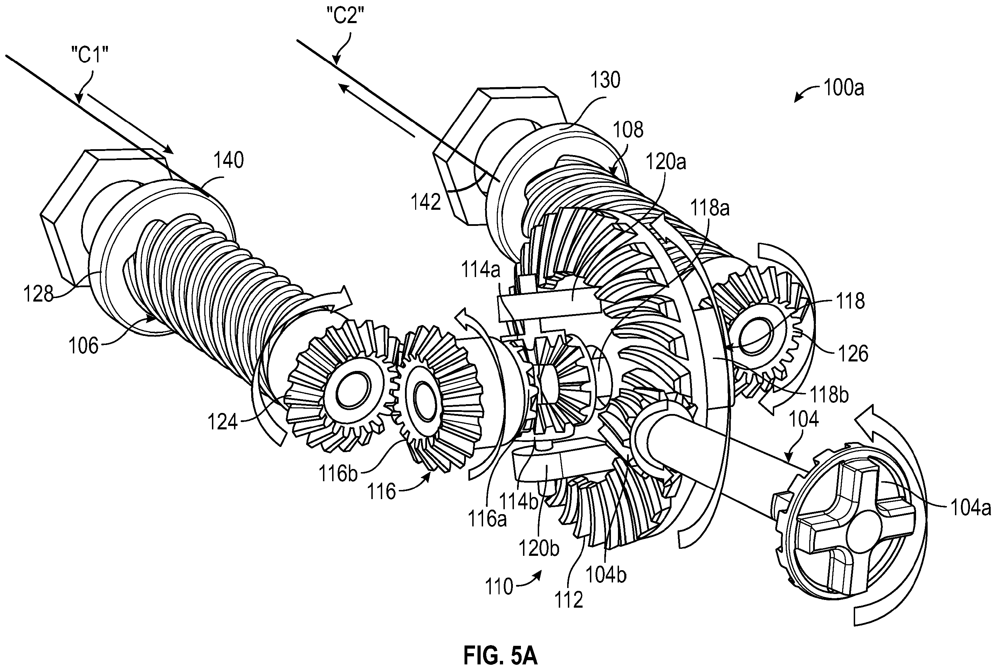

Details about the transmission assembly 100 of the surgical instrument 10 will now be described with reference to FIGS. 3-5B. The transmission assembly 100 includes a first transmission 100a (FIG. 5A) and a second transmission 100b (FIG. 5B) each disposed within the housing 102 and nested with one another, or located in axial alignment with one another (e.g., first transmission 100a being located distal of second transmission 100b or first transmission 100a being located proximal of second transmission 100b).

The first transmission 100a includes a rotatable first input shaft 104, first and second output shafts 106, 108 each configured to rotate in response to a rotation of the first input shaft 104, and a first differential gear mechanism 110 operably coupling the first input shaft 104 to the first and second output shafts 106, 108 and the first and second output shafts 106, 108 to one another. The first input shaft 104 has a proximal end portion 104a configured to be drivingly coupled to a corresponding drive member or shaft (not shown) of the instrument drive unit 20 (FIG. 2), and a distal end portion having a pinion gear 104b non-rotationally fixed thereto. The pinion gear 104b is in meshing engagement with a ring gear 112 of the first differential gear mechanism 110 such that the ring gear 112 is rotatable by the pinion gear 104b of the first input shaft 104. The ring gear 112 rotates about an axis that is perpendicular to a rotation axis of the first input shaft 104. In aspects, the ring gear 112 may be a crown wheel.

In addition to the first differential gear mechanism 110 having the ring gear 112, the first differential gear mechanism 110 further includes first and second spider gears 114a, 114b coupled to the ring gear 112, and first and second side axles 116, 118 coupled to the respective spider gears 114a, 114b and the respective output shafts 106, 108. The spider gears 114a, 114b are rotatably supported on respective posts 120a, 120b that are fixed to the ring gear 112 and rotatable therewith about the rotation axis of the ring gear 112. As such, the first and second spider gears 114a, 114b are configured to rotate about their respective axes relative to the ring gear 112 and with the ring gear 112 around the rotation axis of the ring gear 112.

The first and second side axles 116, 118 of the first differential gear mechanism 110 each include opposing first and second gears 116a, 116b, 118a, 118b, such as, for example, bevel gears or crown gears. The first gear 116a of the first side axle 116 is in meshing engagement with both the first and second spider gears 114a, 114b and the second gear 116b of the first side axle 116 is in meshing engagement with a gear 124 (e.g., a bevel gear) of the first output shaft 106. The second side axle 118 extends through a central opening of the ring gear 112. The first gear 118a of the second side axle 118 is in meshing engagement with both the first and second spider gears 114a, 114b and the second gear 118b of the second side axle 118 is in meshing engagement with a gear 126 (e.g., a bevel gear) of the second output shaft 106. Due to the function of the first differential gear mechanism 110, the average of the rotational speed of the first and second output shafts 106, 108 is equal to the input rotational speed of the input drive shaft 104 such that an increase in the speed of one of the output shafts 106 or 108 results in a proportional decrease in the speed of the other of the output shafts 106, 108, the benefit of which will be described in further detail below.

With continued reference to FIG. 5A, the first transmission 100a further includes first and second articulation nuts 128, 130 operably coupled to the respective first and second output shafts 106, 108. The first output shaft 106 may be a left-handed lead screw and the second output shaft 108 may be a right-handed lead screw such that rotation of the output shafts 106, 108 in the same rotational direction is configured to translate the respective articulation nuts 128, 130 therealong in opposing axial directions. For example, as the output shafts 106, 108 rotate in a clockwise direction, the first articulation nut 128 may translate proximally whereas the second articulation nut 130 may translate distally. The first and second articulation cables "C1," "C2" each have a proximal end portion 140, 142 operably coupled to the respective first and second output shafts 106, 108, and a distal end portion 144, 146 (FIG. 4) secured to the end effector 40, such that the first and second articulation cables "C1," "C2" move axially in opposing directions at a different rate from one another to articulate the end effector 40 relative to the shaft 120 to adjust a yaw of the end effector 40.

As illustrated in Table 1 below, it has been discovered that the degree of articulation of the end effector 40 is not linear to the degree of axial movement of the cables "C1-C4"; rather, articulation of the wrist assembly 30 imposes a non-linear curve into the system and to the degree of axial movement of the cables "C1-C4". Consequently, the inside articulation cable (e.g., articulation cable "C1") is translated proximally a greater distance than the outside articulation cable (e.g., articulation cable "C2") is translated distally. That is, the inside articulation cable "C1" traverses a greater axial distance in the same amount of time than does the outside articulation cable "C2". The differential gear mechanism 110 compensates for this difference to allow for the inside articulation cable "C1" to traverse the greater axial distance in the same amount of time thereby reducing stress and strain on the inside articulation cable "C2". Therefore, use of the transmission assembly 100 of this disclosure will result in the articulation cables having a longer lifespan, and articulation of the end effector 40 will be more precise.

**Table 1**

| Joint Angle (deg) | inside Cable (mm) | Outside Cable (mm) | Average Length (mm) | % Change |
|---|---|---|---|---|
| 0 | 7.21313 | 7.21313 | 7.21313 | 0 |
| 15.5 | 6.52968 | 7.85589 | 7.192785 | -0.282852887 |
| 31 | 5.74358 | 8.63665 | 7.190115 | -0.320092238 |
| 46.5 | 4.88954 | 9.45854 | 7.17904 | -0.474854577 |
| 62 | 4.05633 | 10.3072 | 7.181765 | -0.436731082 |

With reference to FIG. 5B, the second transmission 100b (FIG. 5B) of the transmission assembly 100 is substantially similar or identical to the first transmission 100a but is configured to effect a change in pitch of the end effector 40 via translation of a second set of articulation cables "C3," "C4." The second transmission 100b is disposed within the housing 102 (FIG. 3) and may be orientated generally perpendicular to and nested with the first transmission 100a. In aspects, instead of nesting or overlapping the first and second transmissions 100a, 100b, the first and second transmissions 100a, 100b may be positioned in side-by-side relation to one another, or, as mentioned above, may be positioned distal/proximal to one another.

The second transmission 100b includes a rotatable first input shaft 154, first and second output shafts 156, 158 each configured to rotate in response to a rotation of the first input shaft 154, and a second differential gear mechanism 160 operably coupling the first input shaft 154 to the first and second output shafts 156, 158 and the first and second output shafts 156, 158 to one another. The first input shaft 154 has a proximal end portion 154a configured to be drivingly coupled to a corresponding drive member or shaft (not shown) of the instrument drive unit 20 (FIG. 2), and a distal end portion 154b operably coupled to the second differential gear mechanism 160. Since the second differential gear mechanism 160 is substantially similar or identical to the first differential gear mechanism 110, details of the second differential gear mechanism 160 are not provided herein.

The second transmission 100b further includes third and fourth articulation nuts 168, 170 operably coupled to the respective first and second output shafts 156, 158. The first output shaft 156 may be a left-handed lead screw and the second output shaft 158 may be a right-handed lead screw such that rotation of the output shafts 156, 158 in the same rotational direction is configured to translate the respective articulation nuts 168, 170 therealong in opposing axial directions. The articulation cables "C3," "C4" each have a proximal end portion 172, 174 operably coupled to the respective first and second output shafts 156, 158, and a distal end portion (not explicitly shown) secured to the end effector 40, such that the articulation cables "C3," "C4" move axially in opposing directions at a different rate from one another to articulate the end effector 40 relative to the shaft 120 to adjust a pitch of the end effector 40.

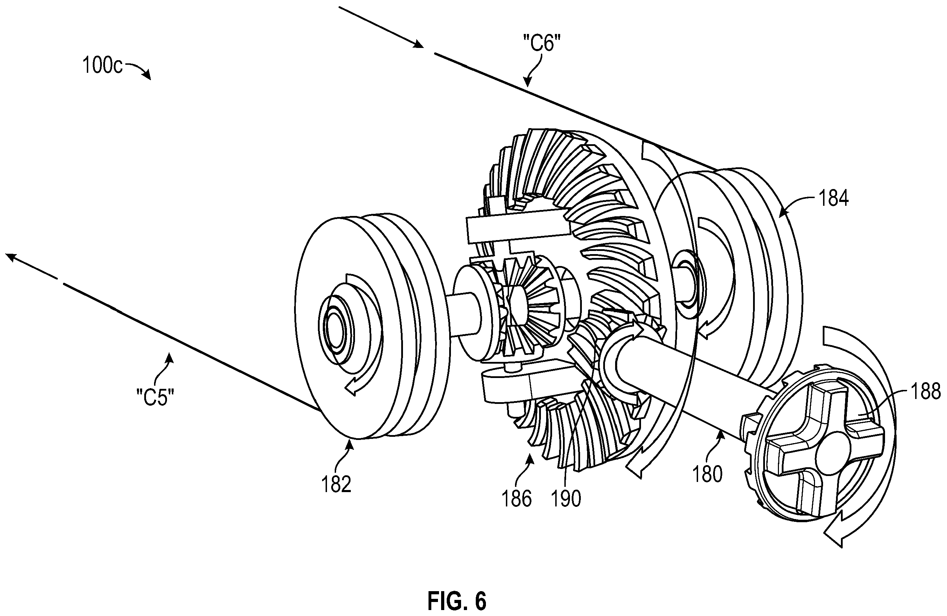

With reference to FIG. 6, another type of transmission 100c is illustrated that can be used in place of or in addition to one or both of the first and second transmissions 100a, 100b. Since the transmission 100c is substantially similar to transmissions 100a, 100b, only those details about the transmission 100c necessary to appreciate the differences from transmissions 100a, 100b will be provided.

The transmission 100c includes a rotatable first input shaft 180, first and second outputs 182, 184 each configured to rotate in response to a rotation of the first input shaft 180, and a third differential gear mechanism 186 operably coupling the first input shaft 180 to the first and second outputs 182, 184 and the first and second outputs 182, 184 to one another. The first input shaft 180 has a proximal end portion 188 configured to be drivingly coupled to a corresponding drive member or shaft (not shown) of the instrument drive unit 20 (FIG. 2), and a distal end portion 190 operably coupled to the third differential gear mechanism 186. Since the third differential gear mechanism 186 is substantially similar or identical to the first and second differential gear mechanisms 110, 160 described above, details of the third differential gear mechanism 180 are not provided herein.

The first and second outputs 182, 184 of the third transmission 100c may be an articulation wheel rotatably supported in the housing 102 and configured to rotate via the third differential gear mechanism 186. Articulation cables "C5," "C6" are fixed to the respective articulation wheels 182, 184 such that the rotation of the articulation wheels 182, 184 axially moves the first and second articulation cables "C5," "C6" in the opposing directions. For example, the articulation cables "C5," "C6" may be secured to the wheels 182, 184 by being wrapped about the wheels 182, 184 in opposing circumferential directions from one another such that rotation of the wheels 182, 184 in the same rotational direction results in the translation of the cables "C5," "C6" in opposing axial directions. In aspects, the outputs 182, 184 may be configured similarly to a capstan or a windlass.

It will be understood that various modifications may be made to the embodiments disclosed herein. Therefore, the above description should not be construed as limiting, but merely as exemplifications of various embodiments. The invention is defined in appended independent claims 1 and 13, preferred embodiments are defined in appended dependent claims.

## Claims

1. A surgical instrument (10) of a surgical robotic system, the surgical instrument comprising:
a housing (102);
a first transmission (100a) disposed within the housing and including:
a rotatable first input shaft (104);
first and second output shafts (106, 108) configured to rotate in response to a rotation of the first input shaft; and
a first differential gear mechanism (110) operably coupling the first input shaft to the first and second output shafts and the first and second output shafts to one another;
a shaft (120) extending distally from the housing;
an end effector (40) pivotably coupled to a distal end portion of the shaft; and
first and second articulation cables (C1, C2) each having a proximal end portion (140, 142) operably coupled to the respective first and second output shafts, and a distal end portion (144, 146) secured to the end effector, such that the first and second articulation cables move axially in opposing directions at a different rate from one another to articulate the end effector relative to the shaft in response to the rotation of the first input shaft.

2. The surgical instrument according to claim 1, wherein the first transmission further includes first and second articulation nuts (128, 130) operably coupled to the respective first and second output shafts and configured to translate therealong in response to a rotation of the respective first and second output shafts, the first and second articulation cables being axially fixed to the respective first and second articulation nuts.

3. The surgical instrument according to claim 1 or claim 2, wherein the first differential gear mechanism includes:
a ring gear (112) operably coupled to the first input shaft such that the ring gear is configured to be rotated by the first input shaft;
first and second spider gears (114a, 114b) coupled to the ring gear such that the first and second spider gears are configured to rotate about a respective axis thereof relative to the ring gear and with the ring gear around a rotation axis of the ring gear; and
first and second side axles (116, 118), the first side axle operably coupled to the first output shaft and the first and second spider gears, and the second side axle operably coupled to the second output shaft and the first and second spider gears; preferably wherein the first side axle has opposing first and second gears (116a, 116b), the first gear is operably coupled to the first and second spider gears and the second gear is operably coupled to the first output shaft.

4. The surgical instrument according to claim 3, wherein the second side axle has opposing first and second gears (118a, 118b), the first gear of the second side axle is operably coupled to the first and second spider gears and the second gear of the second side axle is operably coupled to the second output shaft; and/or wherein the first input shaft has a pinion gear (104b) non-rotationally fixed to a distal end portion thereof, the pinion gear being in meshing engagement with the ring gear.

5. The surgical instrument according to claim 4, wherein the ring gear rotates about an axis that is perpendicular to a rotation axis of the first input shaft.

6. The surgical instrument according to any preceding claim, further comprising:
a second transmission (100b) nested with or positioned adjacent the first transmission and supported in the housing, wherein the second transmission includes:
a rotatable second input shaft (154);
third and fourth output shafts (156, 158) configured to rotate in response to a rotation of the second input shaft; and
a second differential gear mechanism (160) operably coupling the second input shaft to the third and fourth output shafts and the third and fourth output shafts to one another; and
third and fourth articulation cables (C3, C4) each having a proximal end portion (172, 174) operably coupled to the respective third and fourth output shafts, and a distal end portion secured to the end effector, such that the third and fourth articulation cables move axially in opposing directions at a different rate from one another to articulate the end effector relative to the shaft in response to the rotation of the second input shaft; preferably further comprising a wrist assembly (30) movably coupling the end effector to the distal end portion of the shaft, wherein the wrist assembly is configured to allow the end effector to articulate relative to the distal end portion of the shaft to adjust both a pitch and yaw of the end effector.

7. The surgical instrument according to claim 6, wherein the first transmission is configured to change the pitch of the end effector, and the second transmission is configured to change the yaw of the end effector.

8. A surgical robotic system (1), comprising:
a surgical robotic arm (2, 3);
an instrument drive unit (20) configured to be supported on the surgical robotic arm;
and
a surgical instrument according to claim 1 configured to be coupled to and driven by the instrument drive unit,
the housing configured to be attached to the instrument drive unit;
the rotatable first input shaft drivingly coupled to a corresponding drive shaft of the instrument drive unit;

9. The surgical robotic system according to claim 8, wherein the surgical instrument further includes:
a second transmission (100b) nested with or positioned adjacent the first transmission and supported in the housing, wherein the second transmission includes:
a rotatable second input shaft (154);
third and fourth output shafts (156, 158) configured to rotate in response to a rotation of the second input shaft; and
a second differential gear mechanism (160) operably coupling the second input shaft to the third and fourth output shafts and the third and fourth output shafts to one another; and
third and fourth articulation cables (C3, C4) each having a proximal end portion (172, 174) operably coupled to the respective third and fourth output shafts, and a distal end portion secured to the end effector, such that the third and fourth articulation cables move axially in opposing directions at a different rate from one another to articulate the end effector relative to the shaft in response to the rotation of the second input shaft.

10. The surgical robotic system according to claim 9, wherein the surgical instrument further includes a wrist assembly (30) movably coupling the end effector to the distal end portion of the shaft, wherein the wrist assembly is configured to allow the end effector to articulate relative to the distal end portion of the shaft to adjust both the pitch and yaw of the end effector.

11. The surgical robotic system according to claim 9 or claim 10, wherein the first transmission is configured to change the pitch of the end effector, and the second transmission is configured to change the yaw of the end effector.

12. The surgical robotic system according to any of claims 9 to 11, wherein the first transmission further includes first and second articulation nuts (128, 130) operably coupled to the respective first and second output shafts and configured to translate therealong in response to a rotation of the respective first and second output shafts, the first and second articulation cables being axially fixed to the respective first and second articulation nuts.

13. A surgical instrument (10) of a surgical robotic system (1), the surgical instrument comprising:
a housing (102);
a first transmission (100c) disposed within the housing and including:
a rotatable first input shaft (180);
first and second outputs (182, 184) configured to rotate in response to a rotation of the first input shaft; and
a first differential gear mechanism (186) operably coupling the first input shaft to the first and second outputs and the first and second outputs to one another;
a shaft (120) extending distally from the housing;
an end effector (40) pivotably coupled to a distal end portion of the shaft; and
first and second articulation cables (C5, C6) each having a proximal end portion operably coupled to the respective first and second outputs, and a distal end portion secured to the end effector, such that the first and second articulation cables move axially in opposing directions at a different rate from one another to change the pitch or yaw of the end effector relative to the shaft in response to the rotation of the first input shaft wherein each of the first and second outputs includes an articulation wheel (182, 184) configured to rotate via the first differential gear mechanism, the first and second articulation cables (C5, C6) being attached to the respective articulation wheels, such that the rotation of the articulation wheels axially moves the first and second articulation cables in the opposing directions.

14. The surgical instrument according to claim 1, wherein the first output shaft (106) includes a left-handed lead screw and the second output shaft includes a right-handed lead screw; preferably wherein the first transmission further includes first and second articulation nuts (128, 130) operably coupled to the respective left-handed and right-handed lead screws and configured to translate therealong in response to the rotation of the respective left-handed and right-handed lead screws, the first and second articulation cables being axially fixed to the respective first and second articulation nuts.

15. The surgical instrument according to claim 14, further comprising:
a second transmission (100b) nested with or positioned adjacent the first transmission and supported in the housing, wherein the second transmission includes:
a rotatable second input shaft (154);
third and fourth outputs (156, 158) configured to rotate in response to a rotation of the second input shaft; and
a second differential gear mechanism (160) operably coupling the second input shaft to the third and fourth outputs and the third and fourth outputs to one another; and
third and fourth articulation cables (C3, C4) each having a proximal end portion (172, 174) operably coupled to the respective third and fourth outputs, and a distal end portion secured to the end effector, such that the third and fourth articulation cables move axially in opposing directions at a different rate from one another to articulate the end effector relative to the shaft in response to the rotation of the second input shaft.

## Patentansprüche

1. Chirurgisches Instrument (10) eines chirurgischen Robotersystems, das chirurgische Instrument umfassend:
ein Gehäuse (102);
ein erstes Getriebe (100a), das innerhalb des Gehäuses angeordnet ist und einschließt:
eine drehbare erste Eingangswelle (104);
eine erste und eine zweite Ausgangswelle (106, 108), die konfiguriert sind, um sich als Reaktion auf eine Drehung der ersten Eingangswelle zu drehen; und
einen ersten Differentialgetriebemechanismus (110), der die erste Eingangswelle mit der ersten und der zweiten Ausgangswelle und die erste und die zweite Ausgangswelle miteinander wirkkoppelt;
eine Welle (120), die sich von dem Gehäuse distal erstreckt;
einen Endeffektor (40), der mit einem distalen Endabschnitt der Welle schwenkbar gekoppelt ist; und
ein erstes und ein zweites Gelenkkabel (C1, C2), die jedes einen proximalen Endabschnitt (140, 142), der mit der jeweiligen ersten und zweiten Ausgangswelle wirkgekoppelt ist, und einen distalen Endabschnitt (144, 146) aufweisen, der an dem Endeffektor derart gesichert ist, dass sich das erste und das zweite Gelenkkabel mit einer voneinander unterschiedlichen Geschwindigkeit in entgegengesetzte Richtungen axial bewegen, um den Endeffektor als Reaktion auf die Drehung der ersten Eingangswelle relativ zu der Welle gelenkig zu bewegen.

2. Chirurgisches Instrument nach Anspruch 1, wobei das erste Getriebe ferner eine erste und eine zweite Gelenkmutter (128, 130), die mit der jeweiligen ersten und zweiten Ausgangswelle wirkgekoppelt und konfiguriert sind, um sich als Reaktion auf eine Drehung der jeweiligen ersten und zweiten Ausgangswelle daran entlang zu verschieben, einschließt, wobei das erste und das zweite Gelenkkabel an der jeweiligen ersten und zweiten Gelenkmutter axial befestigt sind.

3. Chirurgisches Instrument nach Anspruch 1 oder 2, wobei der erste Differentialgetriebemechanismus einschließt:
ein Tellerrad (112), das derart mit der ersten Eingangswelle wirkgekoppelt ist, dass das Tellerrad konfiguriert ist, um durch die erste Eingangswelle gedreht zu werden;
ein erstes und ein zweites Planetenrad (114a, 114b), die derart mit dem Tellerrad gekoppelt sind, dass das erste und das zweite Planetenrad konfiguriert sind, um sich um eine jeweilige Achse davon relativ zu dem Tellerrad und mit dem Tellerrad um eine Drehachse des Tellerrads zu drehen; und
eine erste und eine zweite Seitenachse (116, 118), wobei die erste Seitenachse mit der ersten Ausgangswelle und dem ersten und dem zweiten Planetenrad wirkgekoppelt ist und die zweite Seitenachse mit der zweiten Ausgangswelle und dem ersten und dem zweiten Planetenrad wirkgekoppelt ist; vorzugsweise wobei die erste Seitenachse ein gegenüberliegendes erstes und zweites Zahnrad (116a, 116b) aufweist, das erste Zahnrad mit dem ersten und dem zweiten Planetenrad wirkgekoppelt ist und das zweite Zahnrad mit der ersten Ausgangswelle wirkgekoppelt ist.

4. Chirurgisches Instrument nach Anspruch 3, wobei die zweite Seitenachse ein gegenüberliegendes erstes und zweites Zahnrad (118a, 118b) aufweist, das erste Zahnrad der zweiten Seitenachse mit dem ersten und dem zweiten Planetenrad wirkgekoppelt ist und das zweite Zahnrad der zweiten Seitenachse mit der zweiten Ausgangswelle wirkgekoppelt ist; und/oder wobei die erste Eingangswelle ein Ritzel (104b) aufweist, das drehfest an einem distalen Endabschnitt davon befestigt ist, wobei das Ritzel mit dem Tellerrad ineinandergreift.

5. Chirurgisches Instrument nach Anspruch 4, wobei sich das Tellerrad um eine Achse, die senkrecht zu einer Drehachse der ersten Eingangswelle ist, dreht.

6. Chirurgisches Instrument nach einem vorstehenden Anspruch, ferner umfassend:
ein zweites Getriebe (100b), das mit dem ersten Getriebe geschachtelt oder angrenzend an diesem positioniert und in dem Gehäuse gelagert ist, wobei das zweite Getriebe einschließt:
eine drehbare zweite Eingangswelle (154);
eine dritte und eine vierte Ausgangswelle (156, 158), die konfiguriert sind, um sich als Reaktion auf eine Drehung der zweiten Eingangswelle zu drehen; und
einen zweiten Differentialgetriebemechanismus (160), der die zweite Eingangswelle mit der dritten und der vierten Ausgangswelle und die dritte und die vierte Ausgangswelle miteinander wirkkoppelt; und
ein drittes und ein viertes Gelenkkabel (C3, C4), die jedes einen proximalen Endabschnitt (172, 174), der mit der jeweiligen dritten und vierten Ausgangswelle wirkgekoppelt ist, und einen distalen Endabschnitt aufweisen, der an dem Endeffektor derart gesichert ist, dass sich das dritte und das vierte Gelenkkabel mit einer voneinander unterschiedlichen Geschwindigkeit in entgegengesetzte Richtungen axial bewegen, um den Endeffektor als Reaktion auf die Drehung der zweiten Eingangswelle relativ zu der Welle gelenkig zu bewegen; vorzugsweise ferner umfassend eine Handgelenkbaugruppe (30), die den Endeffektor mit dem distalen Endabschnitt der Welle bewegbar koppelt, wobei die Handgelenkbaugruppe konfiguriert ist, um dem Endeffektor zu ermöglichen, sich relativ zu dem distalen Endabschnitt der Welle gelenkig zu bewegen, um sowohl ein Nicken als auch eine Gierung des Endeffektors zu justieren.

7. Chirurgisches Instrument nach Anspruch 6, wobei das erste Getriebe konfiguriert ist, um das Nicken des Endeffektors zu ändern, und das zweite Getriebe konfiguriert ist, um die Gierung des Endeffektors zu ändern.

8. Chirurgisches Robotersystem (1), umfassend:
einen chirurgischen Roboterarm (2, 3);
eine Instrumentenantriebseinheit (20), die konfiguriert ist, um auf dem chirurgischen Roboterarm gelagert zu werden; und
ein chirurgisches Instrument nach Anspruch 1, das konfiguriert ist, um mit der Instrumentenantriebseinheit gekoppelt und durch diese angetrieben zu werden,
wobei das Gehäuse konfiguriert ist, um an der Instrumentenantriebseinheit angebracht zu werden;
wobei die drehbare erste Eingangswelle mit einer entsprechenden Antriebswelle der Instrumentenantriebseinheit antriebsmäßig gekoppelt ist;

9. Chirurgisches Robotersystem nach Anspruch 8, wobei das chirurgische Instrument ferner einschließt:
ein zweites Getriebe (100b), das mit dem ersten Getriebe geschachtelt oder angrenzend an diesem positioniert und in dem Gehäuse gelagert ist, wobeidas zweite Getriebe einschließt:
eine drehbare zweite Eingangswelle (154);
die dritte und die vierte Ausgangswelle (156, 158), die konfiguriert sind, um sich als Reaktion auf eine Drehung der zweiten Eingangswelle zu drehen; und
einen zweiten Differentialgetriebemechanismus (160), der die zweite Eingangswelle mit der dritten und der vierten Ausgangswelle und die ditte und die vierte Ausgangswelle miteinander wirkkoppelt; und
das dritte und das vierte Gelenkkabel (C3, C4), die jedes einen proximalen Endabschnitt (172, 174), der mit der jeweiligen dritten und vierten Ausgangswelle wirkgekoppelt ist, und einen distalen Endabschnitt aufweisen, der an dem Endeffektor derart gesichert ist, dass sich das dritte und das vierte Gelenkkabel mit einer voneinander unterschiedlichen Geschwindigkeit in entgegengesetzte Richtungen axial bewegen, um den Endeffektor als Reaktion auf die Drehung der zweiten Eingangswelle relativ zu der Welle gelenkig zu bewegen.

10. Chirurgisches Robotersystem nach Anspruch 9, wobei das chirurgische Instrument ferner eine Handgelenkbaugruppe (30), die den Endeffektor mit dem distalen Endabschnitt der Welle bewegbar koppelt, einschließt, wobei die Handgelenkbaugruppe konfiguriert ist, um dem Endeffektor zu ermöglichen, sich relativ zu dem distalen Endabschnitt der Welle gelenkig zu bewegen, um sowohl das Nicken als auch die Gierung des Endeffektors zu justieren.

11. Chirurgisches Robotersystem nach Anspruch 9 oder 10, wobei das erste Getriebe konfiguriert ist, um das Nicken des Endeffektors zu ändern, und das zweite Getriebe konfiguriert ist, um die Gierung des Endeffektors zu ändern.

12. Chirurgisches Robotersystem nach einem der Ansprüche 9 bis 11, wobei das erste Getriebe ferner eine erste und eine zweite Gelenkmutter (128, 130), die mit der jeweiligen ersten und zweiten Ausgangswelle wirkgekoppelt und konfiguriert sind, um sich als Reaktion auf eine Drehung der jeweiligen ersten und zweiten Ausgangswelle daran entlang zu verschieben, einschließt, wobei das erste und das zweite Gelenkkabel an der jeweiligen ersten und zweiten Gelenkmutter axial befestigt sind.

13. Chirurgisches Instrument (10) eines chirurgischen Robotersystems (1), das chirurgische Instrument umfassend:
ein Gehäuse (102);
ein erstes Getriebe (100c), das innerhalb des Gehäuses angeordnet ist und einschließt:
eine drehbare erste Eingangswelle (180);
einen ersten und einen zweiten Ausgang (182, 184), die konfiguriert sind, um sich als Reaktion auf eine Drehung der ersten Eingangswelle zu drehen; und
einen ersten Differentialgetriebemechanismus (186), der die erste Eingangswelle mit dem ersten und dem zweiten Ausgang und den ersten und den zweiten Ausgang miteinander wirkkoppelt;
eine Welle (120), die sich von dem Gehäuse distal erstreckt;
einen Endeffektor (40), der mit einem distalen Endabschnitt der Welle schwenkbar gekoppelt ist; und
ein erstes und ein zweites Gelenkkabel (C5, C6), die jedes einen proximalen Endabschnitt, der mit dem jeweiligen ersten und zweiten Ausgang wirkgekoppelt ist, und einen distalen Endabschnitt aufweist, der an dem Endeffektor derart gesichert ist, dass sich das erste und das zweite Gelenkkabel in entgegengesetzte Richtungen mit einer voneinander unterschiedlichen Geschwindigkeit axial bewegen, um das Nicken oder die Gierung des Endeffektors relativ zu der Welle als Reaktion auf die Drehung der ersten Eingangswelle zu ändern, wobei jeder des ersten und des zweiten Ausgangs ein Gelenkrad (182, 184), das konfiguriert ist, um sich über den ersten Differentialgetriebemechanismus zu drehen, einschließt, wobei das erste und das zweite Gelenkkabel (C5, C6) an den jeweiligen Gelenkrädern derart angebracht sind, dass die Drehung der Gelenkräder das erste und das zweite Gelenkkabel in die entgegengesetzten Richtungen axial bewegt.

14. Chirurgisches Instrument nach Anspruch 1, wobei die erste Ausgangswelle (106) eine linksgängige Bewegungsschraube einschließt und die zweite Ausgangswelle eine rechtsgängige Bewegungsschraube einschließt; vorzugsweise wobei das erste Getriebe ferner die erste und die zweite Gelenkmutter (128, 130), die mit der jeweiligen linksgängigen und rechtsgängigen Bewegungsschraube wirkgekoppelt und konfiguriert sind, um sich als Reaktion auf die Drehung der jeweiligen linksgängigen und rechtsgängigen Bewegungsschraube daran entlang zu verschieben, einschließt, wobei das erste und das zweite Gelenkkabel axial an der jeweiligen ersten und zweiten Gelenkmutter axial befestigt sind.

15. Chirurgisches Instrument nach Anspruch 14, ferner umfassend:
ein zweites Getriebe (100b), das mit dem ersten Getriebe geschachtelt oder angrenzend an diesem positioniert und in dem Gehäuse gelagert ist, wobei das zweite Getriebe einschließt:
eine drehbare zweite Eingangswelle (154);
den dritten und den vierten Ausgang (156, 158), die konfiguriert sind, um sich als Reaktion auf eine Drehung der zweiten Eingangswelle zu drehen; und
einen zweiten Differentialgetriebemechanismus (160), der die zweite Eingangswelle mit dem dritten und dem vierten Ausgang und den dritten und den vierten Ausgang miteinander wirkkoppelt; und
das dritte und das vierte Gelenkkabel (C3, C4), die jedes einen proximalen Endabschnitt (172, 174), der mit dem jeweiligen dritten und vierten Ausgang wirkgekoppelt ist, und einen distalen Endabschnitt aufweisen, der an dem Endeffektor derart gesichert ist, dass sich das dritte und das vierte Gelenkkabel mit einer voneinander unterschiedlichen Geschwindigkeit in entgegengesetzte Richtungen axial bewegen, um den Endeffektor als Reaktion auf die Drehung der zweiten Eingangswelle relativ zu der Welle gelenkig zu bewegen.

## Revendications

1. Instrument chirurgical (10) d'un système robotique chirurgical, l'instrument chirurgical comprenant :
un logement (102) ;
une première transmission (100a) disposée au sein du logement et comportant :
un premier arbre d'entrée rotatif (104) ;
des premier et deuxième arbres de sortie (106, 108) conçus pour se mettre en rotation en réponse à une rotation du premier arbre d'entrée ; et
un premier mécanisme d'engrenage différentiel (110) accouplant fonctionnellement le premier arbre d'entrée aux premier et deuxième arbres de sortie et les premier et deuxième arbres de sortie l'un à l'autre ;
une tige (120) s'étendant de manière distale par rapport au logement ;
un effecteur terminal (40) accouplé de manière pivotante à une partie d'extrémité distale de la tige ; et
des premier et deuxième câbles d'articulation (C1, C2) ayant chacun une partie d'extrémité proximale (140, 142) accouplée fonctionnellement aux premier et deuxième arbres de sortie respectifs, et une partie d'extrémité distale (144, 146) attachée à l'effecteur terminal, de telle sorte que les premier et deuxième câbles d'articulation se déplacent axialement dans des directions opposées à une vitesse différente l'un de l'autre pour articuler l'effecteur terminal par rapport à la tige en réponse à la rotation du premier arbre d'entrée.

2. Instrument chirurgical selon la revendication 1, dans lequel la première transmission comporte en outre des premier et second écrous d'articulation (128, 130) accouplés fonctionnellement aux premier et deuxième arbres de sortie respectifs et conçus pour se déplacer en translation le long de ceux-ci en réponse à une rotation des premier et deuxième arbres de sortie respectifs, les premier et deuxième câbles d'articulation étant fixés axialement aux premier et second écrous d'articulation respectifs.

3. Instrument chirurgical selon la revendication 1 ou la revendication 2, dans lequel le premier mécanisme d'engrenage différentiel comporte :
une couronne dentée (112) accouplée fonctionnellement au premier arbre d'entrée de telle sorte que la couronne dentée est conçue pour être mise en rotation par le premier arbre d'entrée ;
des premier et second engrenages en étoile (114a, 114b) accouplés à la couronne dentée de telle sorte que les premier et second engrenages en étoile sont conçus pour se mettre en rotation autour d'un axe respectif de ceux-ci par rapport à la couronne dentée et avec la couronne dentée autour d'un axe de rotation de la couronne dentée ; et
des premier et second axes mécaniques latéraux (116, 118), le premier axe mécanique latéral étant accouplé fonctionnellement au premier arbre de sortie et aux premier et second engrenages en étoile, et le second axe mécanique latéral étant accouplé fonctionnellement au deuxième arbre de sortie et aux premier et second engrenages en étoile ; de préférence dans lequel le premier axe mécanique latéral a des premier et second engrenages (116a, 116b) opposés, le premier engrenage est accouplé fonctionnellement aux premier et second engrenages en étoile et le second engrenage est accouplé fonctionnellement au premier arbre de sortie.

4. Instrument chirurgical selon la revendication 3, dans lequel le second axe mécanique latéral a des premier et second engrenages (118a, 118b) opposés, le premier engrenage du second axe mécanique latéral est accouplé fonctionnellement aux premier et second engrenages en étoile et le second engrenage du second axe mécanique latéral est accouplé fonctionnellement au deuxième arbre de sortie ; et/ou dans lequel le premier arbre d'entrée a un engrenage à pignons (104b) fixé de manière non rotative à une partie d'extrémité distale de celui-ci, l'engrenage à pignons étant en mise en prise par engrènement avec la couronne dentée.

5. Instrument chirurgical selon la revendication 4, dans lequel la couronne dentée fait une rotation autour d'un axe qui est perpendiculaire à un axe de rotation du premier arbre d'entrée.

6. Instrument chirurgical selon l'une quelconque revendication précédente, comprenant en outre :
une seconde transmission (100b) imbriquée avec, ou positionnée adjacente à, la première transmission et supportée dans le logement, dans lequel la seconde transmission comporte :
un second arbre d'entrée rotatif (154) ;
des troisième et quatrième arbres de sortie (156, 158) conçus pour se mettre en rotation en réponse à une rotation du second arbre d'entrée ; et
un second mécanisme d'engrenage différentiel (160) accouplant fonctionnellement le second arbre d'entrée aux troisième et quatrième arbres de sortie et les troisième et quatrième arbres de sortie l'un à l'autre ; et
des troisième et quatrième câbles d'articulation (C3, C4) ayant chacun une partie d'extrémité proximale (172, 174) accouplée fonctionnellement aux troisième et quatrième arbres de sortie respectifs, et une partie d'extrémité distale attachée à l'effecteur terminal, de telle sorte que les troisième et quatrième câbles d'articulation se déplacent axialement dans des directions opposées à une vitesse différente l'un de l'autre pour articuler l'effecteur terminal par rapport à la tige en réponse à la rotation du second arbre d'entrée ; comprenant en outre de préférence un ensemble poignet (30) accouplant de manière mobile l'effecteur terminal à la partie d'extrémité distale de la tige, dans lequel l'ensemble poignet est conçu pour permettre à l'effecteur terminal de s'articuler par rapport à la partie d'extrémité distale de la tige pour ajuster à la fois un tangage et un lacet de l'effecteur terminal.

7. Instrument chirurgical selon la revendication 6, dans lequel la première transmission est conçue pour changer le tangage de l'effecteur terminal, et la seconde transmission est conçue pour changer le lacet de l'effecteur terminal.

8. Système robotique chirurgical (1), comprenant :
un bras robotique chirurgical (2, 3) ;
une unité d'entraînement d'instrument (20) conçue pour être supportée sur le bras robotique chirurgical ; et
un instrument chirurgical selon la revendication 1 conçu pour être accouplé à, et entraîné par, l'unité d'entraînement d'instrument,
le logement conçu pour être fixé à l'unité d'entraînement d'instrument ;
le premier arbre d'entrée rotatif accouplé par entraînement à un arbre d'entraînement correspondant de l'unité d'entraînement d'instrument ;

9. Système robotique chirurgical selon la revendication 8, dans lequel l'instrument chirurgical comporte en outre :
une seconde transmission (100b) imbriquée avec, ou positionnée adjacente à, la première transmission et supportée dans le logement, dans lequel la seconde transmission comporte :
un second arbre d'entrée rotatif (154) ;
des troisième et quatrième arbres de sortie (156, 158) conçus pour se mettre en rotation en réponse à une rotation du second arbre d'entrée ; et
un second mécanisme d'engrenage différentiel (160) accouplant fonctionnellement le second arbre d'entrée aux troisième et quatrième arbres de sortie et les troisième et quatrième arbres de sortie l'un à l'autre ; et
des troisième et quatrième câbles d'articulation (C3, C4) ayant chacun une partie d'extrémité proximale (172, 174) accouplée fonctionnellement aux troisième et quatrième arbres de sortie respectifs, et une partie d'extrémité distale attachée à l'effecteur terminal, de telle sorte que les troisième et quatrième câbles d'articulation se déplacent axialement dans des directions opposées à une vitesse différente l'un de l'autre pour articuler l'effecteur terminal par rapport à la tige en réponse à la rotation du second arbre d'entrée.

10. Système robotique chirurgical selon la revendication 9, dans lequel l'instrument chirurgical comporte en outre un ensemble poignet (30) accouplant de manière mobile l'effecteur terminal à la partie d'extrémité distale de la tige, dans lequel l'ensemble poignet est conçu pour permettre à l'effecteur terminal de s'articuler par rapport à la partie d'extrémité distale de la tige pour ajuster à la fois le tangage et le lacet de l'effecteur terminal.

11. Système robotique chirurgical selon la revendication 9 ou la revendication 10, dans lequel la première transmission est conçue pour changer le tangage de l'effecteur terminal, et la seconde transmission est conçue pour changer le lacet de l'effecteur terminal.

12. Système robotique chirurgical selon l'une quelconque des revendications 9 à 11, dans lequel la première transmission comporte en outre des premier et second écrous d'articulation (128, 130) accouplés fonctionnellement aux premier et deuxième arbres de sortie respectifs et conçus pour se déplacer en translation le long de ceux-ci en réponse à une rotation des premier et deuxième arbres de sortie respectifs, les premier et deuxième câbles d'articulation étant fixés axialement aux premier et second écrous d'articulation respectifs.

13. Instrument chirurgical (10) d'un système robotique chirurgical (1), l'instrument chirurgical comprenant :
un logement (102) ;
une première transmission (100c) disposée au sein du logement et comportant :
un premier arbre d'entrée rotatif (180) ;
des première et seconde sorties (182, 184) conçues pour se mettre en rotation en réponse à une rotation du premier arbre d'entrée ; et
un premier mécanisme d'engrenage différentiel (186) accouplant fonctionnellement le premier arbre d'entrée aux première et seconde sorties et les première et seconde sorties l'une à l'autre ;
une tige (120) s'étendant de manière distale par rapport au logement ;
un effecteur terminal (40) accouplé de manière pivotante à une partie d'extrémité distale de la tige ; et
des premier et deuxième câbles d'articulation (C5, C6) ayant chacun une partie d'extrémité proximale accouplée fonctionnellement aux première et seconde sorties respectives, et une partie d'extrémité distale attachée à l'effecteur terminal, de telle sorte que les premier et deuxième câbles d'articulation se déplacent axialement dans des directions opposées à une vitesse différente l'un de l'autre pour changer le tangage ou le lacet de l'effecteur terminal par rapport à la tige en réponse à la rotation du premier arbre d'entrée dans lequel chacune des première et seconde sorties comporte une roue d'articulation (182, 184) conçue pour se mettre en rotation par l'intermédiaire du premier mécanisme d'engrenage différentiel, les premier et deuxième câbles d'articulation (C5, C6) étant fixés aux roues d'articulation respectives, de telle sorte que la rotation des roues d'articulation déplace axialement les premier et deuxième câbles d'articulation dans les directions opposées.

14. Instrument chirurgical selon la revendication 1, dans lequel le premier arbre de sortie (106) comporte une vis-mère à pas à gauche et le deuxième arbre de sortie comporte une vis-mère à pas à droite ; de préférence dans lequel la première transmission comporte en outre des premier et second écrous d'articulation (128, 130) accouplés fonctionnellement aux vis-mères à pas à gauche et à pas à droite respectives et conçus pour se déplacer en translation le long de celles-ci en réponse à la rotation des vis-mères à pas à gauche et à pas à droite respectives, les premier et deuxième câbles d'articulation étant fixés axialement aux premier et second écrous d'articulation respectifs.

15. Instrument chirurgical selon la revendication 14, comprenant en outre :
une seconde transmission (100b) imbriquée avec, ou positionnée adjacente à, la première transmission et supportée dans le logement, dans lequel la seconde transmission comporte :
un second arbre d'entrée rotatif (154) ;
des troisième et quatrième sorties (156, 158) conçues pour se mettre en rotation en réponse à une rotation du second arbre d'entrée ; et
un second mécanisme d'engrenage différentiel (160) accouplant fonctionnellement le second arbre d'entrée aux troisième et quatrième sorties et les troisième et quatrième sorties l'une à l'autre ; et
des troisième et quatrième câbles d'articulation (C3, C4) ayant chacun une partie d'extrémité proximale (172, 174) accouplée fonctionnellement aux troisième et quatrième sorties respectives, et une partie d'extrémité distale attachée à l'effecteur terminal, de telle sorte que les troisième et quatrième câbles d'articulation se déplacent axialement dans des directions opposées à une vitesse différente l'un de l'autre pour articuler l'effecteur terminal par rapport à la tige en réponse à la rotation du second arbre d'entrée.
